# EUROPÄISCHE PATENTSCHRIFT

(11) **EP 1 853 308 B1**
(45) Veröffentlichungstag und Bekanntmachung des Hinweises auf die Patenterteilung: **29.02.2012**
(21) Anmeldenummer: 05818637.0
(22) Anmeldetag: 13.12.2005
(51) Int. Cl.: A61K 39/205, A61K 9/00

(54) **PHARMAZEUTISCHES, ORAL APPLIZIERBARES PRÄPARAT ZUR BEHANDLUNG VON FISCHEN, HERSTELLUNGSVERFAHREN FÜR DIESES UND SEINE VERWENDUNG**
PHARMACEUTICAL PREPARATION THAT CAN BE ADMINISTERED ORALLY FOR TREATING FISH, PRODUCTION METHOD FOR SAID PREPARATION AND USE OF THE LATTER
PREPARATION PHARMACEUTIQUE A USAGE ORAL DESTINEE A TRAITER DES POISSONS, PROCEDE DE PRODUCTION ET UTILISATION ASSOCIES

(30) Priorität: 02.03.2005 DE 102005010288
(43) Veröffentlichungstag der Anmeldung: 14.11.2007
(73) Patentinhaber: RIEMSER Arzneimittel AG, 17493 Greifswald - Insel Riems (DE)
(72) Erfinder: ADELMANN, Malte, 18059 Rostock (DE); FICHTNER, Dieter, 17943 Greifswald - Riemser Ort (DE); LANGE, Bodo, 17493 Greifswald - Riemser Ort (DE); WEITSCHIES, Werner, 17498 Neuenkirchen (DE)
(74) Vertreter: Reinstädler, Diane
(86) Internationale Anmeldenummer: PCT/EP2005/013497
(87) Internationale Veröffentlichungsnummer: WO 2006/092168

(56) Entgegenhaltungen:
- WO-A-01/54514
- WO-A-89/05154
- WO-A-03/086097

## Beschreibung

Die vorliegende Erfindung betrifft ein pharmazeutisches, oral applizierbares Präparat in der Anwendung zur oralen Vakzination von Fischen, insbesondere zur Vakzination der Salmoniden und anderen Fischen mit vergleichbarem Verdauungssystem gegen bakterielle und/oder virale Infektionen, insbesondere gegen VHS und IHN. Die Erfindung betrifft ferner ein Verfahren zur Herstellung des Präparats.

Fischseuchen wie die Virale Hämorrhagische Septikämie (VHS) und die Infektiöse Hämatopoetische Nekrose (IHN) der Salmoniden, zu denen insbesondere Forellen zählen, sind trotz intensiver Eradikationsmaßnahmen, wie die Haltung in nachweislich seuchenfreien Zuchtbetrieben, nach wie vor ein massives Problem für die Fischwirtschaft. Allein in Deutschland wurden in den vergangenen Jahren jährlich 34 bis 80 Neuausbrüche dieser viralen Erkrankungen gemeldet. Bei Infektion eines Bestandes beträgt die Mortalität der Fische bis zu 90 %. Der entstehende ökonomische Schaden durch Neuausbrüche von VHS und IHN ist hoch und beträgt in der Europäischen Union hochgerechnet jährlich zwischen 12 und 36 Millionen Euro. Die Immunprophylaxe ist eine Möglichkeit, Fischkrankheiten gezielt zu bekämpfen. Die Vakzination von Fischen hat sich weltweit noch nicht durchgesetzt, ist aber essentiell für zahlreiche Fischzuchten und Haltungsformen. Jedoch sind die oft kostenintensive Herstellung, die mangelnde Anwenderfreundlichkeit sowie die teilweise geringe Effektivität der Impfstoffe Ursache für den begrenzten Einsatz.

Eine weit verbreitete Methode, Fische zu immunisieren, ist die intraperitoneale Injektion. Hierbei werden die Fische durch Tauchen in eine Betäubungsmittellösung narkotisiert und jedem einzelnen Exemplar die entsprechende Dosis in die Bauchhöhle injiziert. Diese Art der Immunisierung ist gekennzeichnet durch Induktion sehr hoher Schutzraten und gilt weitestgehend als die effektivste. Jedoch ist diese Methode, bedingt durch den immensen Zeitaufwand, ungeeignet für eine Immunisierung großer Populationen sowie für eine extensive Aquakultur nur bedingt anwendbar. Außerdem werden bei dieser Applikationsmethode die Fische großem Stress ausgesetzt und es kann zu Verlusten durch die Betäubung kommen. Ebenfalls sehr kostenintensiv und aufwendig ist die Immunisierung mittels maschinellen Injektionsvorrichtungen. Trotz sehr guter Ergebnisse konnte sich diese Art der Vakzinierung nur bedingt durchsetzen.

Ein weiteres Verfahren zur Vakzination von Fischen stellt die Immersion dar. Dazu zählt die Badimmunisierung. Hierbei werden die Impflinge für einen bestimmten Zeitraum in einem Tauchbad immunisiert. Diese Methode wurde mit inaktivierten Bakterien und attenuierten Viren getestet. Auch hier sind die Fische immensem Stress ausgesetzt. Die Schutzraten sind nur in geringem Maße akzeptabel, so dass dieses Verfahren bisher kaum praktische Anwendung findet. Ebenfalls eine Art der Immersion ist das Sprühverfahren. Hierbei werden die Impflinge mit einer Antigenlösung mäanderförmig besprüht. Die Aufnahme der Vakzine erfolgt über die Schleimhaut und zeigt je nach Art des Antigens sehr unterschiedliche Erfolge. Auch dieses Verfahren hat nur geringe praktische Bedeutung.

Weiterhin findet man in der Literatur Arbeiten zur Immunisierung von Fischen mittels DNA-Vakzination. Ein DNA-Plasmid, welches für bestimmte Proteine mit Antigencharakter von zum Beispiel Viren (VHS) codiert, wird dazu den Fischen intramuskulär injiziert. Die Ergebnisse zeigen eine hohe Schutzrate, allerdings wird diese Methode derzeit ausschließlich im experimentellen Maßstab angewendet.

Die orale Vakzination ist die derzeit einfachste Methode, eine große Anzahl von Fischen zu immunisieren [Irie, T., Watarai, S., Kodama, H.: Humoral immune response of carp (Cyprinus carpio) induced by oral immunization with liposome-entrapped antigen. Developmental & Comperative Immunology 27 (2002), 413-421]. Dazu werden die flüssigen oder lyophilisierten Impfstoffe entweder dem Futter beigemischt oder zu einer oral applizierbaren festen Arzneiform verarbeitet. Wesentliche Vorteile der oralen Immunisierung sind die Anwendbarkeit für große Mengen von Fischen und für Fische jeden Alters, die stressfreie und anwenderfreundliche Applikation. Bisher erprobte oral applizierbare Arzneiformen zeigen eine vergleichsweise geringe Wirksamkeit und erfordern einen komplizierten und kostenaufwändigen Herstellungsprozess.

Bei der oralen Anwendung ist eine Passage des Impfstoffes durch den bei zahlreichen Fischspezies sauren gastralen Trakt (pH 2-4) notwendig, wobei die Vakzine durch die Wirkung der Magensäure inaktiviert werden kann und somit kein ausreichender Schutz induziert wird. Bisher entwickelte Arzneiformen zur oralen Immunisierung von Fischen sind so konzipiert, dass die Pellets unzersetzt den Magen passieren und den Wirkstoff im Darm des Fisches freisetzen. So ist bekannt, den Impfstoff durch einen magensaftresistenten Überzug vor der Inaktivierung durch die Magensäure zu schützen, wobei es jedoch zu einem erheblichen Titerabfall beim Prozess des Überziehens kommen kann. Verantwortlich dafür sind die teilweise sehr hohen Temperaturen und die Tatsache, dass viele magensaftresistente Überzugsmaterialien selbst organische Säuren sind, die eine Inaktivierung des Impfstoffes an der Grenzfläche nach sich ziehen können. Weiterhin kann - bei festgestellten Verweilzeiten solcher Präparate im Magen von bis zu 24 h - nicht gesichert werden, dass diese Arzneiform die Vakzine vollständig schützt. Eine vollständige Freisetzung der Vakzine im Darm ist ebenfalls fraglich, da bei überzogenen Arzneiformen die Liberation der Vakzine verzögert erfolgen kann. Zudem ist die Herstellung der magensaftresistenten Überzüge sehr kostenaufwändig.

Weiterhin gibt es Versuche, den Impfstoff in ein Lipid (Hartfett) einzuarbeiten und die mittels Tropfverfahren hergestellten Präparate oral zu verabreichen. Durch enzymatische Verdauungsprozesse sollte es zu einer Freisetzung der Vakzine im Darm der Fische kommen. Nähere Untersuchungen zeigen jedoch, dass dieses Lipid vom Fisch nicht oder unvollständig verdaut wird und dadurch die Liberation des Impfstoffes aus der Arzneiform verhindert wird.

DE 197 41 114 A1 beschreibt ein Präparat zur oralen Immunisierung von Regenbogenforellen. Der Impfstoff wird in eine Mischung aus Laktose und Stärke und gegebenenfalls einem Bindemittel (Gelatine oder andere gebräuchliche Substanzen) eingearbeitet und mit einem magensaftresistenten Matrixbildner (zum Beispiel einer makromolekularem Celluloseverbindung oder einem Ionenaustauscher) durch Wirbelschicht- oder Siebpressgranulation granuliert. Der Matrixbildner soll eine vorzeitige Freigabe im sauren Milieu verhindern. Das Granulat wird zu Tabletten verpresst und im Wirbelschichtverfahren zur Realisierung der Wasser- und Säurestabilität mit magensaftresistenten Copolymerisaten der Acrylsäure überzogen.

In DE 16 17 300 OS ist eine orale, doppelt verkapselte magensaftresistente Arzneiform zur Humanimmunisierung beschrieben, die das attenuierte Adeno-Virus vor einer Inaktivierung während des Herstellungsprozesses schützt. Hierzu wird der Antigen-haltige Kern der Tablette mit einer inerten Pressbeschichtung aus Laktose, Magnesiumstearat und mikrokristalliner Cellulose versehen, die einen Schutz der Viren während des Überziehens mit einem magensaftresistenten Copolymer (Celluloseacetathydrogenphthalat) gewährleistet.

DD 259 13 A1 beschreibt eine oral verfügbare Arzneiform zur Applikation von Lebend- und Tot-Impfstoffen bei Fischen. Das Präparat umfasst einen Vakzinewirkstoff, insbesondere eine Vakzine gegen VHS, und Laktose als Füllstoff, eingebettet in ein Fett oder fettartiges Material. Die Herstellung erfolgt durch Suspendierung des Vakzinewirkstoffs in die auf 25 bis 27 °C erwärmte lipophile Trägermasse und anschließendem Eintropfen der Suspension in eine indifferente Flüssigkeit (Ethanol-Wasser-Gemisch). Die lipophile Trägermasse verhindert eine Freisetzung des Wirkstoffs im Magen und des oberen Darmrohrs.

WO 03/086097 A und WO 01/54514 A beschreiben jeweils ein pharmazeutisches, oral applizierbares Präparat, das insbesondere auch zur Behandlung von Fischen geeignet ist und zur Modulation einer physiologischen Reaktion oder Veranlassung einer Immunantwort dient. Die offenbarte Zusammensetzung enthält zumindest einen pharmakologischen Wirkstoff, der unter anderem ein Vakzinewirkstoff sein kann, und weist ferner eine neutralisierende Komponente zur Neutralisierung im Darm auf. Das Präparat enthält einen magensaftresistenten Überzug oder ist eine Retard-Formulierung und zerfällt somit im Wesentlichen im Darm.

Alle hier aufgeführten Druckschriften beschreiben Arzneiformen, in denen die Vakzine vor dem inaktivierenden Effekt der Magensäure durch Einbettung des Impfstoffs und/oder magensaftresistente Überzüge geschützt wird. Grundsätzlich passiert dabei das Präparat den Magen unzersetzt und zerfällt im Darm. Die möglichen Nachteile wurden oben vorstehend bereits aufgezeigt.

Aufgabe der vorliegenden Erfindung ist es, ein kostengünstig herstellbares, oral applizierbares Präparat zur Fischbehandlung, insbesondere zur Fischimpfung bereitzustellen, das eine verlustfreie Passage der Vakzine durch den gastralen Trakt der Fische sichert. Das Antigen soll im Fisch vollständig freigesetzt werden und somit einen hinreichenden Schutz bei einer Belastungsinfektion induzieren.

Diese Aufgabe wird durch ein pharmazeutisches, oral applizierbares Präparat in der Anwendung zur oralen Vakzination von Fischen, insbesondere zur Vakzination von Salmoniden gegen bakterielle und/oder virale Infektionen, gelöst, das zumindest einen pharmakologischen Wirkstoff, zumindest eine basische Zusatzkomponente, und zumindest eine Trägerkomponente enthält, wobei die Formulierung des Präparats so beschaffen ist, dass dieses im Magen des zu immunisierenden Fisches zumindest zu 80 %, vorzugsweise praktisch vollständig bereits zerfällt. Diese erfindungsgemäße Formulierung zeichnet sich durch ein neuartiges Freisetzungssystem aus, das im Unterschied zu bekannten Konzepten nicht anstrebt, den Zerfall der Arzneiform im Magen zu verhindern, sondern im Gegenteil den möglichst vollständigen Zerfall bereits im Magen der Fische bewirkt, wobei neben dem Wirkstoff auch die mindestens eine basische Zusatzkomponente im Magen freigesetzt wird und dort eine die Magensäure (HCl) neutralisierende und adsorbierende Wirkung entfaltet. Durch die so entstehende lokale und temporäre Neutralisierung wird der pharmakologische Wirkstoff während seiner Passage durch den Magen vor der inaktivierenden Wirkung der Magensäure geschützt. Zudem haben Untersuchungen an so behandelten Fischen ergeben, dass die Passagedauer des Präparats durch den gastralen Trakt des Tieres aufgrund des frühzeitigen Zerfalls wesentlich verkürzt wird. Während herkömmliche, magensaftresistente Präparate eine Verweildauer im Magen von bis zu 24 Stunden aufweisen, konnte nach oraler Einnahme des erfindungsgemäßen Präparates dieses bereits nach etwa 4 Stunden nicht mehr im Magen nachgewiesen werden, das heißt, spätestens nach 4 Stunden erreicht die zerfallene Arzneiform vollständig den Darmtrakt. Insgesamt wird somit durch beide Effekte - neutralisierende Wirkung und verkürzte Passagedauer - eine verlustfreie Passage des pharmakologischen Wirkstoffs durch den Magentrakt gesichert.

Es ist bevorzugt vorgesehen, einen Anteil der zumindest einen basischen Zusatzkomponente in dem Präparat so zu bemessen, dass beim Zerfall des Präparats im Magen des zu immunisierenden Fisches sich zumindest in der Umgebung des zerfallenen Präparats ein pH-Wert im Bereich von 6,0 bis 8,5, insbesondere von 7,0 bis 7,5 einstellt. Unter diesen weitestgehend neutralen Bedingungen sind die meisten säurelabilen Wirkstoffe, insbesondere Antigen-haltige Vakzine stabil.

Als basische Zusatzkomponenten kommen insbesondere Lewisbasen in Frage, das heißt Substanzen, die in wässrigem Milieu unter Freisetzung von OH⁻-Ionen dissoziieren. Vorzugsweise werden pharmakologisch akzeptable anorganische Hydroxide eingesetzt, insbesondere Metallhydroxide oder Mischungen von solchen. Es ist jedoch auch denkbar, andere Protonen-bindende Basen einzusetzen.

Es ist ein besonderer Vorteil des erfindungsgemäßen Präparats, dass die zur Einbettung des Wirkstoffs notwendigen Hilfsstoffe (insbesondere die matrixbildende Trägerkomponente) zur Verarbeitung nicht erwärmt werden müssen. So erlaubt die erfindungsgemäße Formulierung eine Herstellung der zu verabreichenden Pellets durch Kaltextrusion. Hierunter wird eine Extrusion ohne Wärmezufuhr oder sogar mit Kühlung verstanden, das heißt eine Verarbeitung bei Raumtemperatur oder tieferen Temperaturen. Auf diese Weise kommt der wärmeempfindliche Wirkstoff (insbesondere die Vakzine) während des gesamten Herstellungsprozesses nicht mit erhöhten Temperaturen in Kontakt, wodurch seine partielle Inaktivierung vermieden wird. Im Gegensatz hierzu führen die Beschichtungsverfahren der herkömmlichen Präparate mit einer säureresistenten Schicht stets zu gewissen Erwärmungen und somit zu einem unerwünschten Titerabfall. Das bevorzugte Herstellungsverfahren durch Kaltextrusion erfordert nur einen relativ geringen materiellen, zeitlichen und personellen Aufwand.

Entsprechend dem bevorzugten Verarbeitungsverfahren wird vorzugsweise eine extrudierbare Trägerkomponente verwendet, die bei der Verarbeitungstemperatur (insbesondere Raumtemperatur) eine hochviskose, insbesondere zähflüssige bis wachsartige Konsistenz aufweist. Nach einer bevorzugten Ausgestaltung wird als Trägerkomponente ein hochmolekulares natürliches oder synthetisches Polymer eingesetzt, insbesondere ein Polyether (wie Polyethylenglykol) oder Gelatine. Mischungen von diesen kommen ebenfalls in Frage.

Das erfindungsgemäße Präparat entfaltet seine vorteilhafte Wirkung vor allem bei wärme- und säureempfindlichen pharmakologischen Wirkstoffen. Nach einer besonders bevorzugten Ausgestaltung handelt es sich daher dabei um zumindest einen Vakzinewirkstoff, der mindestens einen Lebend- oder Tot-Impfstoff umfasst, insbesondere inaktivierte Bakterien und/oder attenuierte Viren. Entsprechend der bevorzugten Verwendung des Präparats zur Immunisierung von Fischen, insbesondere von Salmoniden, gegen die Virale Hämorrhagische Septikämie (VHS) und/oder die Infektiöse Haematopoetische Nekrose (IHN) enthält das Präparat einen attenuierten Erreger dieser Infektionen, die beide zur Familie der Rhabdo-Viren zählen.

Selbstverständlich kann das Präparat weitere Hilfsstoffe und/oder einen (säurelabilen) Überzug umfassen. Insbesondere kann das Präparat und/oder der Überzug einen Farbstoff zur besseren Akzeptanz für Fische enthalten. Dazu stehen beispielsweise verschiedenfarbige Eisenoxide zur Verfügung, die unter anderem in der Lebensmittelindustrie Verwendung finden. Die Farbe der Extrudate kann zur Abstimmung für unterschiedliche Fischarten variiert werden. Des Weiteren können die Extrudate mit zerkleinertem Fischfutter vermischt werden, um so eine bessere Aufnahme zu gewährleisten. Eine gewisse, zeitlich begrenzte Wasserstabilität wurde durch die Wahl und Zusammensetzung der Hilfsstoffe erzielt.

Eine besonders bevorzugte Formulierung der neu entwickelten Arzneiform ist ein Extrudat, das die Bestandteile Viruslyophilisat (Wirkstoff/Vakzine), Polyethylenglykol (matrixbildende Trägerkomponente), Aluminiumhydroxid und Magnesiumhydroxid (basische Komponenten), Eisenoxid E 171 (Farbstoff) und Talkum (Überzug) enthält.

Weitere bevorzugte Ausgestaltungen der Erfindung ergeben sich aus den übrigen, in den Unteransprüchen genannten Merkmalen.

Die Erfindung wird nachfolgend in einem Ausführungsbeispiel erläutert.

### Herstellung des Präparats

Es wurden 19,9 g Polyethylenglykol (PEG) 1000 und 13,2 g Polyethylenglykol 1550 geschmolzen, für 2 h bei -20 °C eingefroren und in 50 ml flüssigem Stickstoff mit Mörser und Pistill zerkleinert. 8,3 g VHS-Lyophilisat mit einem Virustiter von 10⁵ KID/10mg wurden mit jeweils 3,3 g der neutralisierenden und adsorbierenden Basen Mg(OH)₂ und Al(OH)₃ und ca. 1 g eines Lebensmittelfarbstoffs E171 Eisenoxid gemischt und anteilmäßig in das zerkleinerte Polyethylenglykol eingearbeitet. Das Gemisch wurde mit einem selbst hergestellten Extruder manuell extrudiert und mit ebenfalls gefärbtem Talkum überzogen. Die entstehenden Fresskörper (Extrudate) waren ca. 3 mm lang und besaßen einen Durchmesser von ca. 1,7 mm. Der Titer der Oralvakzine betrug 10^{4,5} KID/g.

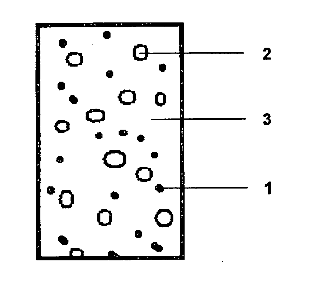

Die Figur zeigt in einem vergrößerten Ausschnitt den schematischen Aufbau des so hergestellten extrudierten Präparats. Darin ist mit 1 der pharmakologische Wirkstoff, das heißt der lyophilisierte VHS-Vakzinewirkstoff bezeichnet und mit 2 die basischen Komponenten Mg(OH)₂/Al(OH)₃. Der VHS-Vakzinewirkstoff 1 sowie die basischen Komponenten 2 sind von einer Matrix 3 des PEG-Trägers (PEG 1000/1500) umhüllt.

### Anwendungsversuch

Die Extrudate wurden dreimal täglich über einen Zeitraum von 72 h verfüttert. Dazu wurde die entsprechende Menge mit 2 g kommerziellem Fischfutter gemischt. 6 Wochen nach der Immunisierung erfolgte eine Infektion mit virulentem VHS-Virus. Bei den oral immunisierten Fischen verendeten nach der Belastungsinfektion lediglich 13,5 %. In der Kontrollgruppe von Fischen, die mit einem Placebo-Präparat ohne Virus behandelt wurden, betrug die Mortalität 70 %. Die Mortalitätsraten der beiden Gruppen unterscheiden sich somit signifikant bei einer Irrtumswahrscheinlichkeit von 5 %.

Die hier vorgestellte Erfindung eignet sich besonders zur Herstellung von Präparaten für die orale Applikation von säurelabilen Viren und Arzneistoffen bei Fischen. Diese Arzneiform bietet den Vorteil, dass durch den Herstellungsprozess die Konzentration oder Aktivität der Wirkstoffe nicht beeinträchtigt wird. Es kann gesichert werden, dass eine Inaktivierung der Wirkstoffe durch das saure Milieu des Magens verhindert wird und eine vollständige Freisetzung des Impfstoffs im Fisch erfolgt. Die Extrudate lassen sich großtechnisch mit geringem materiellen, zeitlichen und personellen Aufwand herstellen. Diese Oralpräparate sind für eine Massenvakzination großer Fischbestände geeignet.

## Patentansprüche

1. Pharmazeutisches, oral applizierbares Präparat in der Anwendung zur oralen Vakzination von Fischen, enthaltend
(a) zumindest einen pharmakologischen Wirkstoff,
(b) zumindest eine basische Zusatzkomponente, und
(c) zumindest eine Trägerkomponente,
**dadurch gekennzeichnet, dass** die Formulierung des Präparats so beschaffen ist, dass dieses im Magen des zu immunisierenden Fisches zumindest zu 80 % zerfällt.

2. Pharmazeutisches Präparat nach Anspruch 1,
**dadurch gekennzeichnet, dass**
die Formulierung des Präparats so beschaffen ist, dass es weitestgehend vollständig im Magen des zu immunisierenden Fisches zerfällt.

3. Pharmazeutisches Präparat nach Anspruch 1 oder 2,
**dadurch gekennzeichnet, dass**
ein Anteil der zumindest einen basischen Zusatzkomponente in dem Präparat so bemessen ist, dass beim Zerfall des Präparats im Magen des zu immunisierenden Fisches sich zumindest lokal ein pH-Wert im Bereich von 6,0 bis 8,5, insbesondere von 7,0 bis 7,5 einstellt.

4. Pharmazeutisches Präparat nach einem der vorhergehenden Ansprüche,
**dadurch gekennzeichnet, dass**
die zumindest eine basische Zusatzkomponente mindestens ein anorganisches Hydroxid ist, insbesondere mindestens ein Metallhydroxid.

5. Pharmazeutisches Präparat nach einem der vorhergehenden Ansprüche,
**dadurch gekennzeichnet, dass**
die zumindest eine Trägerkomponente eine kaltextrudierbare, hochviskose Konsistenz aufweist.

6. Pharmazeutisches Präparat nach einem der vorhergehenden Ansprüche,
**dadurch gekennzeichnet, dass**
die zumindest eine Trägersubstanz ein hochmolekulares, natürliches oder synthetisches Polymer ist, insbesondere ein Polyether, vorzugsweise ein Polyethylenglykol, oder Gelatine oder eine Mischung von solchen ist.

7. Pharmazeutisches Präparat nach einem der vorhergehenden Ansprüche,
**dadurch gekennzeichnet, dass**
das Präparat durch Kaltextrusion hergestellt ist.

8. Pharmazeutisches Präparat nach einem der vorhergehenden Ansprüche,
**dadurch gekennzeichnet, dass**
der zumindest eine pharmakologische Wirkstoff zumindest ein Vakzinewirkstoff ist.

9. Pharmazeutisches Präparat nach Anspruch 8,
**dadurch gekennzeichnet, dass**
der zumindest eine Vakzinewirkstoff mindestens einen Lebend- oder Tot-Impfstoff umfasst, insbesondere inaktivierte Bakterien und/oder attenuierte Viren.

10. Pharmazeutisches Präparat nach Anspruch 8 oder 9,
**dadurch gekennzeichnet, dass**
der zumindest eine Vakzinewirkstoff einen attenuierten Rhabdo-Virus umfasst, insbesondere den Erreger der Viralen Hämorrhagischen Septikämie (VHS) und/oder der Infektiösen Hämatopoetischen Nekrose (IHN).

11. Pharmazeutisches Präparat nach einem der vorhergehenden Ansprüche,
**dadurch gekennzeichnet, dass**
das Präparat weitere Hilfsstoffe umfasst und/oder einen Überzug aufweist.

12. Pharmazeutisches Präparat nach einem der vorhergehenden Ansprüche zur oralen Vakzination von Salmoniden gegen bakterielle und/oder virale Infektionen.

13. Pharmazeutisches Präparat nach Anspruch 12 zur Vakzination von Fischen gegen die Virale Hämorrhagische Septikämie (VHS) und/oder die Infektiöse Hämatopoetische Nekrose (IHN).

14. Verfahren zur Herstellung eines pharmazeutischen, oral applizierbaren Präparats in der Anwendung zur oralen Vakzination von Fischen nach einem der Ansprüche 1 bis 13, wobei eine Masse, enthaltend zumindest einen pharmakologischen Wirkstoff, zumindest eine basische Zusatzkomponente, und zumindest eine Trägerkomponente, hergestellt wird und durch Kaltextrusion zu Fresskörpern verarbeitet wird.

15. Verfahren nach Anspruch 14,
**dadurch gekennzeichnet, dass**
die Fresskörper mit einem Überzug versehen werden, insbesondere einem Talkum- und/oder Farbstoff-haltigen Überzug.

## Claims

1. Pharmaceutical preparation for oral administration in the use for the oral vaccination of fish, containing
(a) at least one pharmacological active substance,
(b) at least one basic additional component, and
(c) at least one carrier component,
**characterized in that** the formulation of the preparation is such that the preparation disintegrates to at least 80% in the stomach of the fish to be immunized.

2. Pharmaceutical preparation according to Claim 1, **characterized in that** the formulation of the preparation is such that the preparation disintegrates virtually completely in the stomach of the fish to be immunized.

3. Pharmaceutical preparation according to Claim 1 or 2, **characterized in that** an amount of the at least one basic additional component in the preparation is such that upon disintegration of the preparation in the stomach of the fish to be immunized a pH in the range of from 6.0 to 8.5, in particular of from 7.0 to 7.5, is established at least locally.

4. Pharmaceutical preparation according to one of the preceding claims, **characterized in that** the at least one basic additional component is at least one inorganic hydroxide, in particular at least one metal hydroxide.

5. Pharmaceutical preparation according to one of the preceding claims, **characterized in that** the at least one carrier component has a cold-extrudable, highly-viscous consistency.

6. Pharmaceutical preparation according to one of the preceding claims, **characterized in that** the at least one carrier substance is a natural or synthetic high-molecular-weight polymer, in particular a polyether, preferably a polyethylene glycol, or gelatin, or a mixture of those.

7. Pharmaceutical preparation according to one of the preceding claims, **characterized in that** the preparation has been prepared by cold-extrusion.

8. Pharmaceutical preparation according to one of the preceding claims, **characterized in that** the at least one pharmacological active substance is at least one vaccine active substance.

9. Pharmaceutical preparation according to Claim 8, **characterized in that** the at least one vaccine active substance comprises at least one live vaccine or inactivated vaccine, in particular inactivated bacteria and/or attenuated viruses.

10. Pharmaceutical preparation according to Claim 8 or 9, **characterized in that** the at least one vaccine active substance comprises an attenuated rhabdovirus, in particular the causative organism of viral haemorrhagic septicaemia (VHS) and/or infectious haematopoietic necrosis (IHN).

11. Pharmaceutical preparation according to one of the preceding claims, **characterized in that** the preparation comprises further adjuvants and/or includes a coating.

12. Pharmaceutical preparation according to one of the preceding claims for the oral vaccination of salmonids against bacterial and/or viral infections.

13. Pharmaceutical preparation according to Claim 12 for the vaccination of fish against viral haemorrhagic septicaemia (VHS) and/or infectious haematopoietic necrosis (IHN).

14. Process for the preparation of a pharmaceutical preparation for oral administration in the use for the oral vaccination of fish according to one of Claims 1 to 13, where a composition containing at least one pharmacological active substance, at least one basic additional component and at least one carrier component is prepared and processed by cold-extrusion to give objects capable of being eaten by animals.

15. Process according to Claim 14, **characterized in that** the objects capable of being eaten by animals are provided with a coating, in particular a talc- and/or colorant-containing coating.

## Revendications

1. Préparation pharmaceutique pouvant être administrée par voie orale, dans l'utilisation pour la vaccination orale de poissons, contenant
(a) au moins une substance active pharmacologique,
(b) au moins un composant additif basique, et
(c) au moins un composant véhicule,
**caractérisée en ce que** la formulation de la préparation doit être réalisée de manière que celle-ci se décompose à raison d'au moins 80 % dans l'estomac du poisson à immuniser.

2. Préparation pharmaceutique selon la revendication 1, **caractérisée en ce que** la formulation de la préparation doit être réalisée de manière que celle-ci se décompose dans la plus large mesure totalement dans l'estomac du poisson à immuniser.

3. Préparation pharmaceutique selon la revendication 1 ou 2, **caractérisée en ce qu'**une proportion dudit au moins un composant additif basique dans la préparation doit être choisie de manière que lors de la décomposition de la préparation dans l'estomac du poisson à immuniser, au moins localement s'ajuste un pH dans l'intervalle de 6,0 à 8,5, en particulier de 7,0 à 7,5.

4. Préparation pharmaceutique selon l'une quelconque des revendications précédentes, **caractérisée en ce que** ledit au moins un composant additif basique est au moins un hydroxyde inorganique, en particulier au moins un hydroxyde de métal.

5. Préparation pharmaceutique selon l'une quelconque des revendications précédentes, **caractérisée en ce que** ledit au moins un composant véhicule présente une consistance très visqueuse, extrudable à froid.

6. Préparation pharmaceutique selon l'une quelconque des revendications précédentes, **caractérisée en ce que** ladite au moins une substance véhicule est un polymère de masse moléculaire élevée, naturel ou synthétique, en particulier un polyéther, de préférence un polyéthylèneglycol, ou la gélatine ou un mélange de ceux-ci.

7. Préparation pharmaceutique selon l'une quelconque des revendications précédentes, **caractérisée en ce que** la préparation est produite par extrusion à froid.

8. Préparation pharmaceutique selon l'une quelconque des revendications précédentes, **caractérisée en ce que** ladite au moins une substance active pharmacologique est au moins une substance active de vaccination.

9. Préparation pharmaceutique selon la revendication 8, **caractérisée en ce que** ladite au moins une substance active de vaccination comprend au moins un vaccin vivant ou mort, en particulier des bactéries inactivées et/ou des virus atténués.

10. Préparation pharmaceutique selon la revendication 8 ou 9, **caractérisée en ce qu'**au moins une substance active de vaccination comprend un rhabdovirus atténué, en particulier l'agent pathogène de la septicémie hémorragique virale (SHV) et/ou de la nécrose hématopoïétique infectieuse (NHI).

11. Préparation pharmaceutique selon l'une quelconque des revendications précédentes, **caractérisée en ce que** la préparation comprend d'autres adjuvants et/ou comporte un enrobage.

12. Préparation pharmaceutique selon l'une quelconque des revendications précédentes, destinée à la vaccination orale de salmonidés contre des infections bactériennes et/ou virales.

13. Préparation pharmaceutique selon la revendication 12, destinée à la vaccination de poissons contre la septicémie hémorragique virale (SHV) et/ou la nécrose hématopoïétique infectieuse (NHI).

14. Procédé pour la fabrication d'une préparation pharmaceutique pouvant être administrée par voie orale, dans l'utilisation pour la vaccination orale de poissons, selon l'une quelconque des revendications 1 à 13, dans lequel on prépare une masse contenant au moins une substance active pharmacologique, au moins un composant additif basique et au moins un composant véhicule, et on la transforme par extrusion à froid en corps alimentaires.

15. Procédé selon la revendication 14, **caractérisé en ce que** les corps alimentaires sont munis d'un enrobage, en particulier d'un enrobage contenant du talc et/ou un colorant.
